(19) Europäisches Patentamt
European Patent Office
Office européen des brevets

(11) EP 2 714 705 B1

(12) # FASCICULE DE BREVET EUROPEEN

(45) Date de publication et mention de la délivrance du brevet:
**22.04.2015 Bulletin 2015/17**

(21) Numéro de dépôt: **12724633.8**

(22) Date de dépôt: **31.05.2012**

(51) Int Cl.:
***C07H 5/10*** (2006.01) ***A61K 8/60*** (2006.01)
***A61K 31/7016*** (2006.01) ***A61Q 19/00*** (2006.01)

(86) Numéro de dépôt international:
**PCT/EP2012/060211**

(87) Numéro de publication internationale:
**WO 2012/163997 (06.12.2012 Gazette 2012/49)**

(54) **SUCROSES OCTASULFATES DE MAGNESIUM, LEUR PREPARATION ET LEURS APPLICATIONS PHARMACEUTIQUES ET COSMETIQUES**

SACCHAROSE-OCTASULFATE AUS MAGNESIUM, HERSTELLUNGSVERFAHREN DAFÜR SOWIE IHRE PHARMAZEUTISCHE UND KOSMETISCHE VERWENDUNG

SUCROSE OCTASULFATES OF MAGNESIUM, PREPARATION METHOD THEREOF AND PHARMACEUTICAL AND COSMETIC USES OF SAME

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **31.05.2011 FR 1154752**

(43) Date de publication de la demande:
**09.04.2014 Bulletin 2014/15**

(73) Titulaire: **Pierre Fabre Dermo-Cosmétique**
**92100 Boulogne-Billancourt (FR)**

(72) Inventeurs:
- **JEULIN, Séverine**
**200040 Shanghai (CN)**
- **HERNANDEZ-PIGEON, Hélène**
**F-31270 Cugnaux (FR)**
- **AGUILAR, Luc**
**F-31750 Escalquens (FR)**

(74) Mandataire: **Regimbeau**
**20, rue de Chazelles**
**75847 Paris Cedex 17 (FR)**

(56) Documents cités:
**EP-A1- 0 640 346**
**WO-A2-2006/017752**
**FR-A1- 2 916 355**
**US-A- 5 618 798**
**US-A- 5 908 836**

EP 2 714 705 B1

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

## Description

La présente invention concerne des sucroses octasulfates de magnésium, leur procédé de préparation et leur utilisation dans le domaine pharmaceutique et/ou cosmétique.

Les oligosaccharides sont des glucides dont l'hydrolyse produit uniquement des sucres simples (oses). Ils sont constitués par l'union d'au moins deux molécules de sucres simples. Parmi les oligosaccharides, on trouve le sucrose formé par la condensation de deux oses : une molécule de glucose et une molécule de fructose.

Les oligosaccharides sulfatés sont connus dans la littérature et possèdent de multiples activités biologiques, cosmétiques et/ou thérapeutiques.

Par exemple, la demande WO2006/017752 décrit une méthode de traitement des inflammations des voies aériennes utilisant des oligosaccharides sulfatés comme substances actives. Parmi ceux ci se trouve, en particulier, un oligosaccharide issu de la condensation du glucose et du fructose et totalement sulfaté.

US5767104 décrit des oligosaccharides sulfatés, principalement le sucrose octasulfate d'aluminium, dans le traitement de l'alopécie.

Par ailleurs, le sucrose octasulfate est utilisé comme principe actif dans le traitement des ulcères gastriques pour ses propriétés réparatrices/cicatrisantes. FR 2 646 604 décrit des formulations de sucrose octasulfate d'aluminium, ou sucralfate, aux propriétés anti-inflammatoires et cicatrisantes, destinées au traitement de plaies ou autres inflammations ulcéreuses. WO1994/0047 décrit une méthode de traitement des lésions et/ou inflammations de l'appareil digestif par administration d'un sel de sucrose sulfaté, plus particulièrement le sucrose octasulfate de potassium ou de sodium.

FR 1390007 décrit l'utilisation par voie topique de formulations contenant du sucralfate en association avec du sulfate de cuivre et de zinc comme régénérants tissulaires, cicatrisants et apaisants.

Enfin, EP0230023 ainsi que FR2916355 décrivent l'utilisation d'oligosaccharides polysulfatés, plus particulièrement du sucrose octasulfate de potassium, comme agent guérissant les blessures. D'autres utilisations de tels composés sont également décrites dans US 5 908 836, US 4 581 221 et EP 0 640 346.

Les problèmes de cicatrisation se retrouvent dans un grand nombre de pathologies, d'accidents ou font suite aux opérations chirurgicales. Il existe donc en permanence un besoin de compositions alternatives permettant d'améliorer la cicatrisation et/ou de l'accélérer.

Dans la présente invention, les inventeurs ont obtenu de nouveaux composés ayant des propriétés réparatrices, antimicrobiennes et anti-radicalaires surprenantes. En effet, de façon surprenante, il a été montré que ces composés induisent la migration des kératinocytes, la synthèse d'acide hyaluronique, la différenciation des kératinocytes et la synthèse de peptides antimicrobiens au contraire d'autres sucroses octasulfates métalliques. Ainsi, ces composés permettent la réparation de la peau, la guérison de blessures et favorisent la cicatrisation, et ce, d'une manière très efficace. Par ailleurs, les inventeurs ont montré que ces composés pouvaient aussi être utilisés en cosmétique pour maintenir le confort et la beauté de la peau

La Figure 1 représente la quantité relative des ARNm de loricrine produits par des kératinocytes traités pendant 72h avec du sucrose octasulfate de magnésium à 30 μM (SOS-Mg), du sucrose octasulfate de sodium à 30 μM (SOS-Na) ou du sucrose octasulfate de magnésium à 30 μM (SOS-Mg) par rapport au témoin négatif

La Figure 2 représente la quantité relative des ARNm de Padi 1 produits par des kératinocytes traités pendant 72h avec du sucrose octasulfate de magnésium à 30 μM (SOS-Mg), du sucrose octasulfate de sodium à 30 μM (SOS-Na) ou du sucrose octasulfate de magnésium à 30 μM (SOS-Mg) par rapport au témoin négatif.

La Figure 3 représente la quantité relative des ARNm de hBD2 produits par des kératinocytes traités pendant 72h avec du sucrose octasulfate de magnésium à 30 μM (SOS-Mg), du sucrose octasulfate de sodium à 30 μM (SOS-Na) ou du sucrose octasulfate de magnésium à 30 μM (SOS-Mg) par rapport au témoin négatif

La présente invention porte en particulier sur des composés de formule générale I suivante,

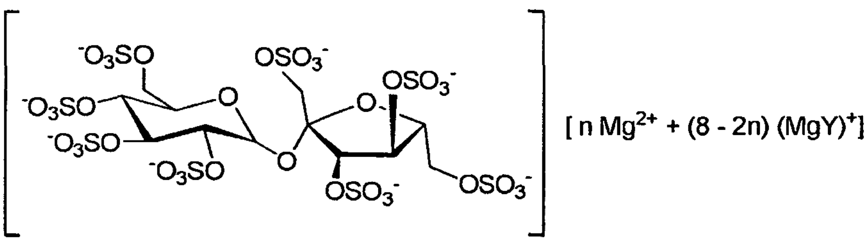

Formule I

dans laquelle :

$0 \leq n \leq 4$
n est un nombre entier
Y représente OH, Cl, Br, I, $NO_3$, $C_6H_5O_7$, $CH_3CO_2$, $CF_3CO_2$ ou $-OCH_3$.

Dans un mode de réalisation de l'invention, le composé de formule I selon l'invention est tel que n=4. Ce composé est représenté par la formule II suivante.

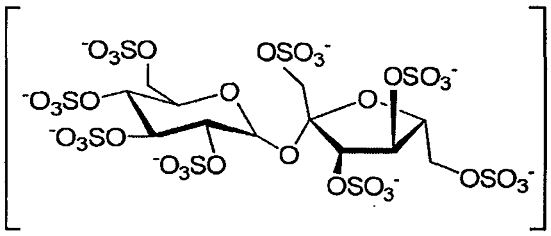

$[4\ Mg^{2+}]$

Formule II

Dans un autre mode de réalisation de l'invention, le composé de formule I selon l'invention est tel que n=3. Ce composé est représenté par la formule III suivante.

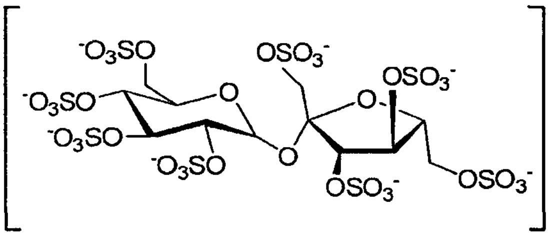

$[3\ Mg^{2+} + 2\ (MgY)^{+}]$

Formule III

Dans un autre mode de réalisation de l'invention, le composé de formule I selon l'invention est tel que n=2. Ce composé est représenté par la formule IV suivante.

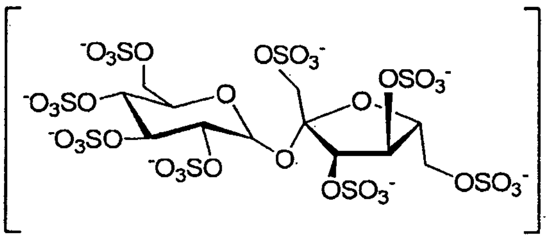

$[2\ Mg^{2+} + 4\ (MgY)^{+}]$

Formule IV

Dans un autre mode de réalisation de l'invention, le composé de formule I selon l'invention est tel que n=1. Ce composé est représenté par la formule V suivante.

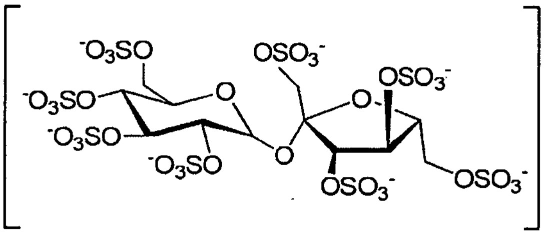

$[Mg^{2+} + 6\ (MgY)^{+}]$

Formule V

Dans un autre mode de réalisation de l'invention, le composé de formule I selon l'invention est tel que n=0. Ce composé est représenté par la formule VI suivante.

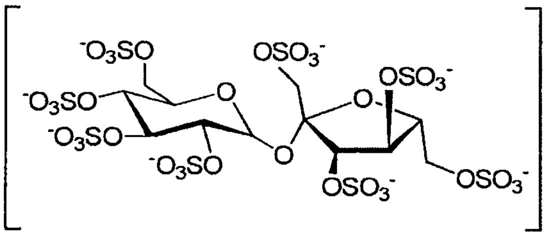

$[8\ (MgY)^{+}]$

Formule VI

Dans ce mode de réalisation de l'invention, le composé de formule VI correspond à un composé de formule I dans lequel n est égal à 0.

Dans toutes les formules III à VI ci-dessus, Y représente OH, Cl, Br, I, $NO_3$, $C_6H_5O_7$, $CH_3CO_2$, $CF_3CO_2$ ou $-OCH_3$.

Préférentiellement, le composé de formule I selon l'invention est tel que n=4.

Dans un mode de réalisation, le composé selon l'invention est sous forme hydratée.

La présente invention porte en outre sur un procédé de préparation des composés selon l'invention.

En particulier, l'invention porte sur un procédé de préparation des composés de formule I selon l'invention caractérisé en ce qu'il comprend les étapes suivantes :

a. mise en contact de la forme acide du sucrose octasulfate en solution, préférentiellement en solution aqueuse telle que l'eau, avec un sel de magnésium pour former du sucrose octasulfate de magnésium, et
b. récupération du sucrose octasulfate de magnésium ainsi formé.

Le procédé selon l'invention comprend donc une première étape a. dans laquelle la forme acide du sucrose octasulfate en solution est mise en contact avec un sel de magnésium.

Par sucrose octasulfate sous forme acide on comprend du sucrose octasulfate de formule VII suivante :

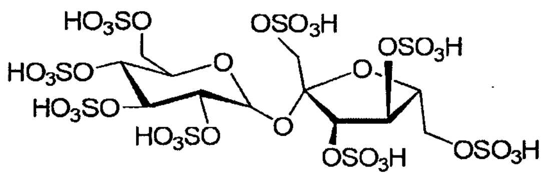

Formule VII

Par « mise en contact » selon l'invention, on entend la mise en présence du sucrose octasulfate sous forme acide et d'un sel de magnésium dans des conditions permettant leur complexation. Préférentiellement, le sel de magnésium est dissout dans la solution de sucrose octasulfate sous forme acide. Les quantités relatives de sucrose octasulfate sous forme acide et du sel de magnésium dans la solution sont choisies de manières à obtenir le composé de formule I souhaité.

Le temps de contact entre le sucrose octasulfate sous forme acide et le sel de magnésium pourra être déterminé

par des tests de routine et dépendra notamment de la nature du sel de magnésium utilisé. Lorsque le sel utilisé est l'hydroxyde de magnésium, le temps de contact sera préférentiellement compris entre 8 et 15 heures.

La mise en contact sera préférentiellement réalisée sous agitation par exemple à l'aide d'un agitateur magnétique.

Le « sel de magnésium» selon l'invention est préférentiellement choisi parmi les sels minéraux de magnésium tels que, par exemple $Mg(OH)_2$, $MgCl_2$, $MgBr_2$, MgIr, $Mg(NO_3)_2$ ou $Mg(BF_4)_2$, ou un sel organique de magnésium, choisi parmi $Mg(CH_3CO_2)_2$, $Mg(CF_3CO_2)_2$, $Mg_3(C_6H_5O_7)_2$ ou $Mg(CH_3O)_2$ Préférentiellement le sel de magnésium selon l'invention est l'hydroxyde de magnésium $Mg(OH)_2$.

Le mélange obtenu après l'étape a. pourra éventuellement être filtré, notamment pour supprimer d'éventuels sels de magnésium restants.

Le procédé selon l'invention comprend une seconde étape b. dans laquelle le sucrose octasulfate de magnésium obtenu à l'étape a. est récupéré.

Par récupération selon l'invention, on entend préférentiellement l'obtention d'un solide de sucrose octasulfate de magnésium, éventuellement un cristal.

Les méthodes permettant de récupérer un tel solide sont bien connue de l'homme du métier par exemple la récupération selon l'invention pourra être réalisée par extraction/précipitation par ajout d'un solvant au mélange obtenu à l'étape a. Alternativement, il est possible de récupérer le solide directement par lyophilisation d'une solution aqueuse obtenue à l'étape a. Dans un mode de réalisation préféré, le solvant est ajouté à la solution aqueuse obtenue à l'étape a. et est mélangé à celle ci. L'ensemble est ensuite décanté. La phase la moins dense contenant le cristal est ensuite récupérée. Cette phase sera préférentiellement lavée à l'eau puis sera être lyophilisée afin d'obtenir le sucrose octasulfate de calcium sous forme solide.

Dans un mode de réalisation, le pH de la solution obtenue à l'étape a. est ajusté à pH basique, par exemple à pH 8.

Afin d'augmenter le rendement de la précipitation et la pureté du composé obtenu, la phase dense obtenue après décantation pourra être dissoute dans de l'eau puis à nouveau précipitée dans le solvant. Cette mise en solution et re-extraction dans le solvant pourra être réalisée autant de fois que nécessaire pour obtenir la pureté et le rendement désiré en sucrose octasulfate de magnésium. Un tel procédé de récupération du sucrose octasulfate de calcium est notamment décrit dans les exemples.

Le solvant sera préférentiellement un solvant organique ou un mélange de solvants organiques tels que par exemple l'acétone, l'éthanol, l'acétate d'éthyle, la méthylisobutylcétone ou la méthyléthylcétone, préférentiellement l'acétone ou l'éthanol..

Les étapes a. et b. sont préférentiellement réalisées à l'obscurité.

La forme acide du sucrose octasulfate en solution utilisée dans l'étape a. du procédé selon l'invention pourra être obtenue de toutes les façons connues de l'homme du métier. Préférentiellement, le sucrose octasulfate sous forme acide sera obtenu à partir d'un sel de sucrose octasulfate.

Dans un mode de réalisation préféré, le sucrose octasulfate sous forme acide est obtenu à partir d'un sel de sucrose octasulfate en réalisant les étapes suivantes :

al. dissolution dans une solution, préférentiellement aqueuse telle que l'eau d'un sel de sucrose octasulfate,
a2. désalification de la solution de sel de sucrose octasulfate ainsi obtenue pour former la forme acide du sucrose octasulfate en solution.

Le « sel de sucrose octasulfate » selon l'invention peut être choisi parmi tous les sels de sucrose octasulfate existants tel que par exemple les sels de métaux alcalins (tel que par exemple le potassium, le sodium, le lithium, etc.). Préférentiellement, le sel de sucrose octasulfate est le sucrose octasulfate de potassium ou le sucrose octasulfate de sodium.

L'étape al. permet l'obtention d'une solution de sel de sucrose octasulfate.

Par « dissolution » du sel de sucrose octasulfate on entend la mise en solution dudit composé. L'homme du métier peut facilement estimer le volume de solution, préférentiellement aqueuse telle que l'eau, à ajouter pour solubiliser totalement la quantité de sel de sucrose octasulfate souhaitée et pour obtenir une solution à la concentration requise (par exemple entre 0,01M et 1M). La dissolution est préférentiellement réalisée par agitation du sel dans une solution aqueuse, telle que l'eau, par exemple grâce à un agitateur magnétique.

Le procédé selon l'invention peut également comprendre une étape a.2 dans laquelle le sel dissout obtenu à l'étape a1. est désalifié.

Par « désalification » on entend la dissociation dans le sel de sucrose octasulfate de la forme acide du sucrose octasulfate et du cation. Cette étape de désalification est suivie de la récupération de la forme acide du sucrose octasulfate. Cette étape peut être réalisée par toutes les techniques connues de l'homme du métier notamment par passage sur une colonne échangeuse d'ions.

Le passage sur une colonne échangeuse d'ions de la solution contenant le sucrose octasulfate acide peut être

effectué plusieurs fois afin d'éliminer l'ensemble des cations qui lui sont originellement associés.

La colonne échangeuse d'ions contient préférentiellement une résine échangeuse de cations. Dans un mode de réalisation de l'invention, la résine échangeuse de cations est une résine de type acide sulfonique (par exemple l'Amberlite™).

Dans un mode de réalisation préféré, le procédé selon l'invention comprendra ou consistera en les étapes suivantes :

a1. dissolution dans une solution d'un sel de sucrose octasulfate,
a2. désalification de la solution de sel de sucrose octasulfate ainsi obtenue pour former la forme acide du sucrose octasulfate en solution.
a. mise en contact de la forme acide du sucrose octasulfate en solution avec un sel de magnésium pour former du sucrose octasulfate de magnésium, et
b. précipitation du sucrose octasulfate de magnésium ainsi formé.

Dans un autre mode de réalisation, le sucrose octasulfate de magnésium est obtenu directement à partir du sucralfate. Le sucralfate est tout d'abord sulfaté, par exemple dans la pyridine puis mis en présence d'une base forte, telle que le ($Mg(OH)_2$) par exemple.

La présente invention porte, en outre, sur des compositions comprenant un ou plusieurs composés selon l'invention et au moins un excipient pharmaceutiquement ou cosmétologiquement acceptable.

L'invention a également pour objet un dispositif médical comprenant un ou plusieurs composés selon l'invention et un excipient pharmaceutiquement ou cosmétologiquement acceptable.

Préférentiellement, la composition selon l'invention ou le dispositif médical selon l'invention ne contiendront qu'un seul type de composés de formule I, préférentiellement le composé de formule II.

«L'excipient» selon l'invention pourra, par exemple, être seuls ou en association un ou plusieurs tensioactifs, un ou plusieurs solvants, un ou plusieurs polymères hydrosolubles, un ou plusieurs épaississants ou gélifiants, un ou plusieurs conservateurs, un ou plusieurs antibactériens, un ou plusieurs antiseptiques, un ou plusieurs agents cicatrisants, un ou plusieurs agents antioxydants, un ou plusieurs agents émollients et/ou hydratants, un ou plusieurs pigments, un ou plusieurs parfums et/ou un ou plusieurs colorants, des agents d'ajustement du pH tels que les sels, des acides, des bases.

Par « pharmaceutiquement et/ou cosmétologiquement acceptable » selon l'invention on se réfère à des entités moléculaires et des compositions qui ne produisent aucun effet adverse, allergique ou autre réaction indésirable quand elles sont administrées à un animal ou un humain.

La composition exacte et la forme de la composition selon l'invention pourra être déterminée par l'homme du métier en fonction de l'utilisation et la voie d'administration envisagées pour la composition.

La composition selon l'invention sera préférentiellement formulée de manière à pouvoir être administrée par voie topique ou orale.

Par «administration par voie topique » selon l'invention on comprend notamment les applications cutanées, les applications buccales (muqueuse buccale), les applications génitales (muqueuse anale, vaginale).

Par « administration par voie orale » selon l'invention on comprend notamment les administrations par ingestion (dont gastriques).

Lorsque la composition est formulée de manière à pouvoir être administrée par voie topique, elle sera préférentiellement sous une forme permettant une application facilité telle que une poudre, un lait, une crème, un baume, une huile, une lotion, un gel, une mouse, un gel moussant, une pommade, d'un spray, une pâte, un patch, etc. Alternativement, lorsque les voies d'administration anale ou vaginale sont envisagées, la composition selon l'invention pourra être sous forme de suppositoire, ovule ou capsule.

Lorsque la composition est formulée de manière à pouvoir être administrée sous forme orale elle sera préférentiellement sous forme d'une gomme, d'une pastille, d'un comprimé, d'un sucre cuit, d'un gel à boire, d'une poudre à dissoudre, d'un pansement gastrique, etc.

Les dosages des composés selon l'invention dans les compositions seront notamment déterminés en fonction de la quantité de substance active nécessaire pour obtenir la réponse thérapeutique et/ou cosmétique désirée, en fonction du mode d'administration envisagé et de la durée du traitement désiré.

Dans un mode de réalisation, la composition selon l'invention présente une teneur en sucrose octasulfate de magnésium selon la formule générale I comprise entre 0,1 et 30 % en poids. Préférentiellement, la composition selon l'invention, par exemple pour une application topique, comprendra entre 0,5 et 7%, encore préférentiellement entre 0,5 et 5% en poids de sucrose octasulfate de calcium.

La composition selon l'invention pourra en outre comprendre au moins un autre principe actif, préférentiellement un autre cicatrisant, un antidouleur, un agent anti radicalaire, un antiseptique et/ou un anti-inflammatoire.

La présente invention porte, en outre, sur des composés selon l'invention ou des compositions selon l'invention

pour leur utilisation en tant que médicaments.

La présente invention porte, en outre, sur l'utilisation d'un composé ou d'une composition selon l'invention pour la fabrication d'un médicament.

La présente invention porte, en outre, sur une méthode de traitement comprenant l'administration à un patient en ayant besoin, d'une dose efficace de composés ou compositions selon l'invention

Préférentiellement, les composés ou compositions selon l'invention sont utilisés pour le traitement de la peau, des muqueuses ou des organes, encore préférentiellement pour favoriser la cicatrisation de la peau, des muqueuses ou des organes et/ou pour les protéger des infections microbiennes et/ou pour lutter contre des infections microbiennes et/ou pour lutter contre l'inflammation.

Par « cicatrisation » selon l'invention, on entend notamment les phénomènes de régénération et de consolidation des tissus ou des organes afin de combler une lésion.

Par « favorisation de la cicatrisation » on entend notamment que la cicatrisation se fait de façon plus rapide et/ou plus efficace (absence ou diminution des cicatrices...) en présence du composé ou d'une composition selon l'invention.

Préférentiellement, l'utilisation selon l'invention concernera la cicatrisation des plaies aigües ou chroniques et des brûlures.

L'expression «plaie aiguë ou chronique » selon l'invention comprend notamment les écorchures, les éraflures, les égratignures, les coupures, les aphtes, les diverses plaies de la sphère buccale, l'acné cicatriciel, les ampoules, les chéilites, l'eczéma, les érythèmes fessiers, les dermatoporoses, les ulcères par exemple gastrique ou de la jambe, les escarres, les plaies du diabétique (en particulier des pieds), les irritations diverses, les dermites ou les cicatrices post chirurgie ou post acte de dermatologie esthétique (laser, épilation, peeling, injection).

Le terme « brulure » selon l'invention comprend les brûlures de toute origine dont les brulures thermiques, mécaniques, chimiques ou dues aux rayonnements. Les brulures selon l'invention peuvent notamment être les radiodermites, les brulures consécutives à des coups de soleil ou dues à la chaleur, les cicatrices de cryothérapie, les cicatrices post chirurgie ou post acte de dermatologie esthétique (laser, épilation, peeling).

Par « infection microbienne » on entend toutes les infections cutanées ou des muqueuses qu'elles soient dues à des bactéries, des levures, des champignons ou des virus. Les compositions selon l'invention peuvent être utilisées dans un but préventif pour éviter l'apparition d'une infection microbienne ou à but thérapeutique pour lutter contre une infection microbienne déjà existante.

Par « inflammation » on entend une réaction défense immunitaire du corps à une agression caractérisée notamment par une rougeur, un gonflement, une sensation de chaleur et de douleur. La présente invention concerne particulièrement le traitement des états inflammatoires de la peau.

La quantité de sucralfate à administrer a un patient dépendra de la pathologie à traiter et du mode d'administration. Par exemple, lorsqu'une application gastrique est envisagée, le sucrose octasulfate de calcium pourra être administré à une dose de 1 à 10 g/jour, préférentiellement de 2 à 6 g/jour.

La présente invention porte, en outre, sur l'utilisation d'un composé ou d'une composition cosmétique selon l'invention pour améliorer l'aspect de la peau.

Par « améliorer l'aspect de la peau» selon l'invention on entend l'amélioration esthétique ou de confort d'un état de la peau et/ou des muqueuses.

La dégradation esthétique de l'état de la peau ou de son confort peut être due par exemple à l'âge, aux conditions extérieures ou aux variations de poids. Par exemple, la composition cosmétique selon l'invention pourra permettre de restaurer la fonction barrière de la peau, l'hydratation de la peau, l'augmentation de son élasticité et/ou de sa tonicité et aussi la réduction des vergetures, de la cellulite ou des rides ainsi que la disparition des taches cutanées.

Alternativement le composé ou la composition selon l'invention pourra être utilisé afin de prévenir le vieillissement cutané.

Les exemples suivants sont donnés à titre illustratif et ne limitent pas la portée de l'invention.

EXEMPLES

Exemple 1 : Préparation du composé de Formule II

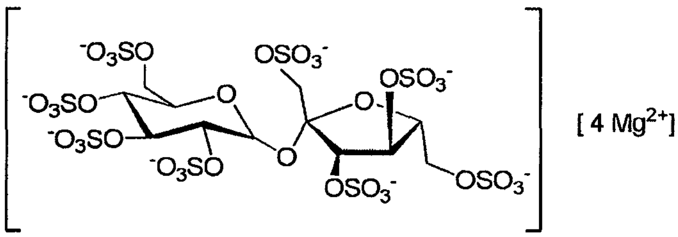

Une solution de sucrose octasulfate de potassium (1,50 g; 1,16 mmol, 1,00 equiv, 99%) dans l'eau (40 ml) est placée dans un ballon de 100 ml. La solution est passée à travers une colonne (Φ 40x500 mm) contenant 250 g de résine échangeuse d'ions d'Amberlite IR 120 H à un débit de 1 ml/min à 0°C. La fraction acide (120 ml ; pH ≤ 1,5) est collectée et neutralisée immédiatement par ajout d'hydroxyde de magnésium à pH = 10,23. Le mélange résultant, est laissé sous agitation pendant une nuit (env. 12 h) à température ambiante et à pH = 9,84.

Le mélange est ensuite filtré et 700 ml d'acétone sont additionnés au filtrat. Le mélange résultant est laissé au repos pour la nuit. Le surnageant est décanté et le sirop restant est dissous dans 15 ml d'eau déminéralisée, et 200 ml d'acétone sont ajoutés à la solution. Le mélange résultant est laissé au repos pendant 3 heures et le surnageant est décanté. Une telle opération est répétée 3 fois. Puis le sirop est dissous dans 30 ml d'eau, et le mélange est lyophilisé. Un solide blanc de sucrose octasulfate de magnésium est obtenu (0,50 g ; 40%).

Toutes ces étapes ont été réalisées à l'abri de la lumière en enveloppant le milieu réactionnel avec du papier d'aluminium.

Caractérisation du composé de formule II obtenu :

Le spectre RMN du composé de formule II obtenu est le suivant: RMN $^1H$ ($D_2O$, 300 MHz, *ppm):* δ: 4,13-4,42 (m, 9 H); 4,52 (m, 1 H); 4,63 (m, 2 H); 5,04 (d, *J* = 8,1 Hz, 1 H); 5,73 (d, *J* = 3,3 Hz, 1 H).

Par ailleurs un dosage du sucrose octasulfate et du magnésium contenu dans le composé II obtenu (SOS-Mg) est effectué. Le même dosage est réalisé dans des échantillons témoins ayant une concentration en Mg ou en sucrose octasulfate connue : le MgCl2 et le sucrose octasulfate de potassium (SOS-K). Le dosage est réalisé de la manière suivante :

Solution standard et préparation des échantillons :

- 5,2 mg de $MgCl_2$ (pureté : 97%) pesés avec précision dans une fiole jaugée de 10 ml et dissous complètement dans une solution aqueuse à 0.05% en TFA, la concentration finale étant de 0,504 mg/ml.
- 12,1 mg de SOS-K (teneur en eau : 8.82%) pesés avec précision dans une fiole jaugée de 10 ml et dissous complètement dans une solution aqueuse à 0.05% en TFA, la concentration finale étant de 1,21 mg/ml.
- 16,1 mg de SOS-Mg pesés avec précision dans une fiole jaugée de 10 ml et dissous complètement dans une solution aqueuse à 0.05% en TFA, la concentration finale étant de 1,61 mg/ml. Un échantillon de 8,0 ml est dilué avec une solution aqueuse à 0,05% en TFA dans une fiole jaugée de 10 ml, pour l'analyse du sucrose octasulfate dans SOS-Mg.

Analyse par HPLC :

Les échantillons SOS-Ca, SOS-K et Cacl2 ainsi obtenus sont caractérisés par HPLC grâce au matériel et conditions suivants : colonne : Atlantis T3 (4,6*100 mm ; 3,0 μm), température de la colonne : 30°C, débit : 0,6 ml/min, volume d'injection : 5 μl pour l'analyse de Mg dans SOS-Mg, 20 μl pour l'analyse de SOS dans SOS-Mg détection : ELSD (température « tube de transfert » = 50°C ; débit gazeux = 2,0 l/min, phase mobile A : 0,05 % TFA/Eau, phase mobile B : 0,05 %/Acétonitrile (gradient : T0 A :100%, T2 A :100%, T5 A :5%, B :95%).

Les résultats obtenus quant à la teneur en magnésium et en sucrose octasulfate sont représentés dans les tableaux 1 et 2 suivants :

Tableau 1 : Estimation de la concentration en Mg de l'échantillon SOS-Mg à partir du standard $MgCl_2$.

| | Concentration (mg/ml) | Concentration en Mg (mg/ml) | Aire du pic/teneur en Mg |
|---|---|---|---|
| $MgCl_2$ | 0,504 | 0,1274 | 1116301 |
| SOS-Mg | 1,61 | - | 1164648 |

Le calcul de la concentration en Mg dans l'échantillon de SOS-Mg permet d'obtenir une valeur de 3,3960 mmol/g.

Tableau 2 : Estimation de la concentration en sucrose octasulfate de l'échantillon SOS-Mg à partir du standard SOS-K.

| | Concentration en SOS (mg/ml) | Aire du pic/teneur en SOS |
|---|---|---|
| SOS-K | 0,1274 | 5322294 |
| SOS-Mg | - | 6637820 |

Le calcul de la concentration en SOS dans l'échantillon de SOS-Mg permet d'obtenir une valeur de 0,8304 mmol/g.

Comme on peut le constater, le ratio magnésium / sucrose octasulfate dans le composé de formule II SOS-Mg obtenu est de 3,3961/0,8304, soit de 4,09 ce qui correspond bien au ratio attendu.

Exemple 2 : Effet du sucrose octasulfate de Mg sur la migration des cellules

La migration des cellules épithéliales est une étape importante du développement et des processus de réparation tissulaire, tels que l'embryogenèse et la cicatrisation.

Les mécanismes d'initiation, de coordination et d'arrêt des mouvements des cellules, ne sont pas complètement élucidés, cependant, le rôle primordial de la migration cellulaire est bien établi.

Lors de la cicatrisation cutanée et dans les affections inflammatoires chroniques dermatologiques, les kératinocytes sont "activés" pour entreprendre le processus de migration. Les cellules voient alors leur phénotype influencé par les interactions avec la matrice extracellulaire d'une part et par les interactions cellules-cellules, d'autre part. Les kératinocytes de l'assise basale des berges d'une plaie, migrent sur la plaie et la recouvrent.

En effet, les kératinocytes sont activés au contact de la fibronectine, du collagène dermique interstitiel (type 1), du collagène IV et de la laminine 5 de la lame basale. Ils sont aussi régulés par certains facteurs de croissance polypeptidiques comme le TGF$\beta$, le TGF$\alpha$ et l'EGF. De plus, des cytokines (IL1, TNF$\alpha$) et chémokines (RANTES et l'IL-8) contribuent aussi à augmenter la vitesse de ré-épithélisation d'une plaie, suite à l'activation kératinocytaire.

Afin d'évaluer l'impact du sucrose octasulfate de magnésium sur la migration cellulaire de lignées de kératinocytes HaCAT, des études ont été réalisées à l'aide du Kit de migration cellulaire Oris Cell Migration Assay (Platypus Technologies). Cette étude a été menée en parallèle avec le sucrose octasulfate de potassium, et le sucrose octasulfate de sodium pour comparaison.

- Matériel biologique

La lignée de kératinocytes utilisée est la lignée de kératinocytes humains HaCaT, spontanément immortalisés Cette lignée est fréquemment citée comme modèle de référence dans la littérature.

-Protocole de migration cellulaire

Le protocole utilisé pour l'étude de la migration cellulaire repose sur l'utilisation d'un kit 96 puits, Oris Cell Migration Assay (Platypus Technologies - TEBU), permettant la miniaturisation et la quantification de ce processus cellulaire.

Le principe de ce test consiste à étudier la migration cellulaire vers le centre du puits de la plaque de 96 puits. Il consiste à placer un stoppeur dans des puits, afin de créer une zone de détection de 2 mm de diamètre. Puis de retirer les stoppeurs une fois que les cellules ont bien adhéré à la surface autour de ceux-ci, et ainsi, de permettre aux cellules de migrer vers la zone de détection. Les plaques sans les stoppeurs et avec les actifs sont mises à incuber à 37°C pendant 24 heures dans du DMEM 0% SVF. On analyse ensuite la quantité de cellules situées dans la zone où il y avait

le stoppeur, afin d'évaluer la migration des cellules. Un cache permet de visualiser et de comptabiliser uniquement les cellules situées dans cette zone. Pour chaque condition, la moyenne de 4 à 8 puits est réalisée.

Les produits testés dans cette étude sont le SOS-Mg obtenu dans l'exemple 1 à 10μM, du SOS-K à 10μM (Sucrose octasulfate de potassium), du SOS-Na à 10μM (Sucrose octasulfate de sodium) et de l'EGF en tant que témoin positif. Un témoin négatif est réalisé dans lequel aucun composé n'est ajouté au milieu de culture (Témoin 0% SVF).

Résultats

L'effet des 3 sucroses a été testé sur la migration des cellules HaCat.

Les résultats sont quantifiés grâce à la formule suivante :

$$\frac{\textit{IF traité}}{\textit{IF témoin 0\% SVF}} \quad X\ 100$$

- en IF (Intensité de Fluorescence, proportionnelle à la quantité des cellules ayant migrées).
- en pourcentage d'activité par rapport au témoin 0% SVF .

Les résultats obtenus sont résumés dans le tableau 3.

Tableau 3 : Effet des 3 sucroses sur la migration des kératinocytes. * $p<0.05$ et *** $p<0.001$ par rapport au témoin 0%SVF.

| | **Témoin** | **EGF** | **SOS-Mg** | **SOS-Na** | **SOS-K** |
|---|---|---|---|---|---|
| | **0% SVF** | **33ng** | **10μM** | **10μM** | **10μM** |
| MOY IF | 5044 ± 1074 | 16138 ± 1548 | 7084 ± 662 | 4988 ± 965 | 5001 ± 835 |
| % activité / témoin | 100 | 320*** | 140* | 99 | 99 |

De ce tableau on peut déduire que :

- L'EGF à 33 ng/ml, contrôle positif de nos expériences, induit la migration (et la prolifération) des kératinocytes de façon très importante et significative.
- A 10 μM, le sucrose octasulfate de potassium et le sucrose octasulfate de sodium n'induisent pas la migration des kératinocytes.
- A 10 μM, le sucrose octasulfate de magnésium (SOS-Mg) induit de façon statistiquement significative la migration des kératinocytes. Cette migration est 1.4 fois plus importante que celle induite par le SOS-Na et le SOS-K.

Exemple 3 : Effet du sucrose octasulfate de Mg sur la différenciation des cellules L'épiderme joue un rôle protecteur majeur en procurant une barrière chimique et mécanique pour le corps. Elle assure le maintien de l'étanchéité, à savoir la fonction barrière cutanée. Les cornéocytes, kératinocytes de la couche cornée, associés à une matrice lipidique, assurent en grande partie cette fonction. Néanmoins les couches plus profondes interviennent dans la mise en place des acteurs de cette fonction. La capacité de différenciation des kératinocytes de l'épiderme va garantir la mise en place d'une barrière de type perméabilité sélective fonctionnelle. Le programme de différenciation des kératinocytes est régulé de manière spatiotemporelle, évoluant des couches les plus profondes de l'épiderme, couche basale la moins différenciée, vers la couche cornée, stade ultime de différenciation des kératinocytes en cornéocytes. D'un point de vue cellulaire et moléculaire, peuvent être principalement observés, la formation des filaments de kératine, la transformation des kératinocytes en cornéocytes ou "cornification", et la mise en place d'un ciment lipidique intercellulaire organisé en structures lamellaires, assurant l'étanchéité et la fonction barrière cutanée.

D'un point de vue protéique, la différenciation épidermique est majoritairement axée sur l'évolution de protéines de structure que sont les kératines et qui contribuent à l'intégrité architecturale de l'épiderme. Leur expression varie en fonction du degré de maturation des kératinocytes. La kératine 1 basique et la kératine 10 acide, sont des marqueurs précoces de la différenciation kératinocytaire, présents dès la couche basale de l'épiderme. L'expression d'autres marqueurs de ce processus biologique, plus tardifs, peut être suivie telle que celle des protéines de l'enveloppe cornée, comme la cornéodesmosine (CDSN), les small prolin rich protein 1 (SPRR1A et SPRR1B), l'involucrine (IVL), ainsi que certaines enzymes majeures à l'origine du pontage des protéines de structure entre elles et avec des lipides kératinocytaires, les transglutaminases, telles que la transglutaminase 1 (TGM1) ou 3.

La formation de la matrice fibreuse présente dans les cornéocytes est initiée au niveau de la transition entre kératinocytes granuleux et les cornéocytes. La loricrine (LOR) est une protéine de structure contenant des résidus glutamine et lysine permettant la fixation avec d'autres protéines de l'enveloppe cornée. Les molécules basiques de filaggrine (FLG) produites à partir de son précurseur la profilaggrine (stockée dans les granules de kératohyaline) s'associent aux filaments de cytokératine, permettant ainsi leur agrégation. La filaggrine peut être ensuite déiminée par les enzymes Peptidyl Arginine Deiminase (PAD) et notamment PAD1 et PAD3. La filaggrine déiminée, acide, se détache alors des filaments intermédiaires avant d'être complètement dégradée, engendrant les acides aminés qui constituent le facteur naturel d'hydratation (FNH).

Parallèlement, la synthèse et le transport de lipides kératinocytaires sont à l'origine du ciment lipidique intercornéocytaire indispensable à la barrière cutanée, dont la formation représente la phase ultime de la différenciation épidermique terminale. Cette matrice lipidique extra-cellulaire fournit la principale barrière aux mouvements transcutanés d'eau et d'électrolytes. Ainsi un certain nombre d'enzymes et de transporteurs lipidiques voient leur expression kératinocytaire augmentée avec la différenciation. Ce ciment résulte d'un équilibre entre trois espèces lipidiques, le cholestérol, les acides gras libres et les céramides. Ces lipides dérivent de glucosylcéramides, sphingomyeline, cholestérol et de phospholipides synthétisés dans les couches épineuses et granuleuses. Ils sont transportés via les corps lamellaires, petites vésicules sécrétoires qui fusionnent dans la couche granuleuse et déversent leur contenu à la jonction stratum granulosum/stratum corneum. En plus de ces précurseurs lipidiques, les corps lamellaires contiennent de nombreuses enzymes incluant des hydrolases lipidiques comme la Sphyngomyélinase acide (aSmase), la beta-glucocerebrosidase (GBA) ou les plospholipases A2 (sPLA2) ainsi que des lipases acides et neutres. Co-délivrés avec les précurseurs lipidiques dans les espaces extracellulaires, ces enzymes convertissent respectivement, la sphingomyeline en céramide, la beta-glucocérébroside en céramide et les phospholipides en acides gras libres et glycérol. La glucosylceramide synthase (UGCG) intervient aussi dans la barrière lipidique de la peau en permettant la synthèse de glucosylceramide. Sa transcription est augmentée pendant le processus de différenciation.

La fonction barrière de la peau inclut aussi une défense contre les micro-organismes. L'épithélium joue un rôle actif dans les défenses innées de l'hôte. Les systèmes antimicrobiens cutanés reposent entre autres sur la présence de certains lipides de surface (acides oléique et palmitoléique du sébum et de certaines protéines constitutives qui sont de plus en plus exprimées en fonction de l'état de différenciation des kératinocytes (RNAse 7, protéinase inhibiteur 3). La RNase 7 et la protéinase inhibiteur 3 (PI3 ou elafin) possèdent des activités antimicrobiennes. Rnase 7 est un membre de la famille des Rnase A et possède une activité antimicrobienne à large spectre. Elle est ainsi capable d'agir contre des bactéries Gram + ou Gram -.

De plus, l'acidification de la surface épidermique joue un rôle important dans la défense antimicrobienne cutanée. La peau agit ainsi non seulement comme une barrière physique, mais aussi comme une barrière chimique. Il existe également une composante adaptative de l'immunité innée reposant sur la sécrétion inductible de peptides antimicrobiens. Ils possèdent des activités antimicrobiennes directes contre des bactéries variées, des virus et des champignons avec la capacité d'inhiber leur croissance. En plus, de par leur action chimiotactique, ces peptides antimicrobiens font le lien entre réponse immunitaire innée et adaptative. Ils jouent un rôle important comme médiateurs de l'inflammation en ayant des effets sur les cellules épithéliales et inflammatoires, en influençant la prolifération cellulaire, la cicatrisation, la production de cytokines, chimiokines et la chimiotaxie. Les peptides antimicrobiens sont généralement synthétisés dans les couches supérieures de la couche épineuse et la couche granuleuse, mais ils sont actifs dans la couche cornée où ils sont libérés. Leur mode d'action consiste à rompre la membrane plasmique des microbes infectieux ou à pénétrer dans le microorganisme afin d'interférer avec le métabolisme intracellulaire. Les peptides antimicrobiens les plus étudiés dans la peau sont les β-défensines et les cathélicidines.

Les β-défensines humaines constituent la classe majeure des peptides antimicrobiens retrouvés dans les épithéliums humains et quatre d'entres elles ont été identifiées dans la peau, hBD 1-4. Bien qu'elles appartiennent à la même famille, elles sont régulées par des voix différentes.

La β-défensine 2 humaine (hBD-2 ou DEFB4), un peptide de 4 kDa se liant à l'héparine est l'un des principaux peptides antimicrobiens cutanés. Seulement bactériostatique face à S. aureus, hBD-2 possède une activité antimicrobienne essentiellement dirigée contre les bactéries à Gram négative.

La β-défensive 3 (hBD-3 ou DEFB103A), un peptide antimicrobien de 5 kDa présente une activité antimicrobienne à large spectre incluant *Staphylococcus aureus*.

L'effet du sucrose octasulfate de magnésium et des sucroses octasulfate de potassium et de sodium sur la régulation de cibles moléculaires impliquées dans les processus de différenciation kératinocytaire a été évalué. Pour cela des kératinocytes normaux humains (NHK) ont été mis en présence des différents produits à tester et l'expression de différents ARN codant des protéines impliquées dans la mise en place des structures protéiques de l'enveloppe cornée, mais aussi dans la synthèse lipidique ou l'expression de peptides antimicrobiens a été mesurée.

- Matériel biologique

La lignée de kératinocytes utilisée est la lignée de kératinocytes humains primaires ou NHKs préparée à partir de lambeaux cutanés issus de déchets opératoires de chirurgie esthétique (diminution mammaire). Les cellules sont cultivées dans du milieu KSFM (Invitrogen) à faible teneur en magnésium (0.1mM) complémenté par 25 μg/mL de BPE (Bovin Pituitary Extract) et 1.5 ng/mL d'EGF (Epithelium Growth Factor).

- Protocole expérimental

Les produits testés sont le SOS-Mg à 10 μM, le SOS-Na à 10 μM, le SOS-K à 10 μM. En parallèle, les expériences sont réalisées avec ajout de $CaCl_2$ à 1,2mM en tant que témoin positif. Un témoin négatif est réalisé dans lequel aucun produit n'est ajouté au milieu de culture.

Les produits à tester sont incubés avec les kératinocytes pendant 72 heures. Les cellules sont ensuite récupérées et une analyse de l'expression de gènes cibles impliqués dans la différenciation épidermique est réalisé par la technologie Quantigène qui permet de quantifier l'expression des ARNm d'intérêts.

Les valeurs obtenues par le Quantigene sont normalisées par rapport à la moyenne géométrique des 2 gènes de référence POL2RA et HPRT. La quantité relative est calculée par rapport au témoin. La régulation de l'expression du gène d'intérêt est prise en compte à partir d'un QR (quantité relative) $\geq$ 1.9 (induction) ou d'un QR $\leq$ 0,5 (inhibition).

Résultats

L'effet des 3 sucroses à différentes concentrations a été testé sur la différenciation des kératinocytes.

Une première étude a été menée sur l'expression de 14 gènes impliqués dans la différentiation cellulaire. Les résultats sont résumés dans le tableau 4. Les valeurs obtenues sont précisées pour les gènes dont l'expression a été modifiée par l'ajout de SOS-Mg, SOS-K ou SOS-Na à 10μM.

Tableau 4 : Quantités relatives (QR) de l'expression d'ARNm en fonction du produit incubé avec les cellules par rapport au témoin négatif

| | Concentration | DEFB103A | CDSN | RNASE7 | UGCG |
|---|---|---|---|---|---|
| CaCl2 | 1.2 nM | 18.7 | 1.3 | 4.5 | 3.1 |
| SOS-Mg | 10 μM | 3.0 | 1.9 | 2.9 | 2.0 |
| SOS-K | 10 μM | 2.3 | 1.5 | 2.4 | 1.8 |
| SOS-Na | 10 μM | 0.9 | 0.7 | 1.6 | 1.1 |

Comme on peut le constater dans ce tableau :

- Le calcium, contrôle positif de nos expériences, a induit l'expression de 3 ARNm (DEFB103A, RNASE7 et UGCG).
- Le SOS-Mg à 10 μM a induit l'expression de 4 ARNm (DEFB103A, RNASE7, CDSN et UGCG). L'activité du SOS-Mg sur l'expression de la cornéodesmosine (CDSN) indique que le SOS-Mg participe à la restauration de la barrière protéique. Son activité sur l'expression de UGCG indique sa participation sur la restauration de la barrière lipidique. Par ailleurs l'induction de la RNASE7 et de DEFB103A prouve les propriétés anti-microbiennes du composé SOS-Mg.
- Le SOS-Na n'a induit l'expression d'aucun ARNm.
- Le SOS-K à 10 μM a induit l'expression de DEFB103A, RNASE7, UGCG et de CDSN de façon moins importante que le SOS-Mg.

Une seconde étude a été réalisée dans les mêmes conditions mais en mesurant l'expression d'un plus grand nombre de gène (64 cibles impliquées dans la différentiation cellulaire) par PCR en temps réel.

Cette étude montre que le SOS-Mg à 30 μM a induit l'expression de 7 ARNm (FLG, IVL, SPRR1A, SPRR1B, LOR, Padi1 et hBD2). Ces inductions prouvent le rôle du SOS-Mg dans l'induction de la différentiation des kératinocytes et donc de la cicatrisation (IVL, SPRR1A, SPRR1B et LOR), dans l'hydratation (Padil) ainsi que ses propriétés antimicrobiennes (hBD2).

Afin de comparer les effets du SOS-Mg, SOS-Na et le SOS-K dans trois fonctions importantes pour la cicatrisation : différentiation cellulaire, hydratation et propriétés antimicrobiennes, l'induction de 3 gènes la *loricrine, Padil et hBD2 (DEFB4),* par le SOS-Mg, SOS-Na et le SOS-K a été comparée. Les résultats sont représentés dans les figures 1 à 3.

On peut constater que la loricrine, Padi 1 et hBD2 (DEFB4) ont été induits de façon plus importante par le SOS-Mg que le SOS-Na et le SOS-K. Le SOS-Mg induit ainsi à la différenciation des kératinocytes et favorise la formation d'une fonction barrière physique et anti-microbienne ainsi que l'hydratation cutanée.

## Revendications

1. Composé de formule générale I

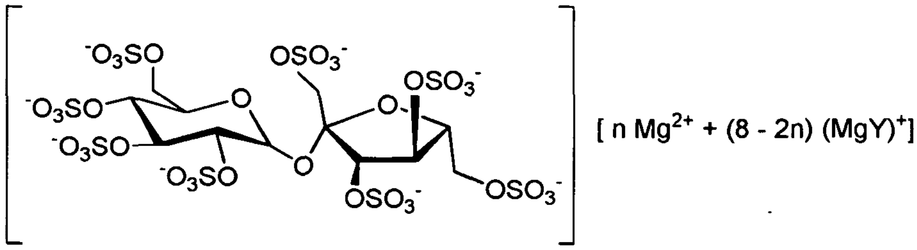

Formule I

dans laquelle :

$0 \leq n \leq 4$
n est un nombre entier
Y représente OH, Cl, Br, I, $NO_3$, $BF_4$, $C_6H_5O_7$, $CH_3CO_2$, $CF_3CO_2$ ou $-OCH_3$, pour son utilisation pour favoriser la cicatrisation et/ou pour la prévention ou le traitement des infections microbiennes.

2. Composé pour son utilisation selon la revendication 1, **caractérisé en ce que** n=4.

3. Composé pour son utilisation selon la revendication 1 ou 2, **caractérisé en ce qu'**il s'agit de la cicatrisation des brûlures et plaies aiguës ou chroniques.

4. Composé pour son utilisation selon la revendication 3 **caractérisé en ce que** les brulures et plaies aigues ou chroniques sont dues à une brûlure par la chaleur ou par un coup de soleil, une radiodermite, une irritation d'origine diverse, une dermite, une écorchure, une éraflure, une égratignure, une coupure, un ulcère par exemple de jambe ou gastrique, un escarre, une plaie du diabétique, un aphte, une plaie de la sphère buccale d'origine diverse, de l'acné cicatriciel, une cicatrice de cryothérapie, une cicatrice post chirurgie ou post acte de dermatologie esthétique, une ampoule, une chéilite, de l'eczéma, un érythème fessier ou une dermatoporose.

5. Composition pharmaceutique comprenant au moins un composé tel que définit dans la revendication 1 ou 2, et au moins un excipient pharmaceutiquement acceptable, pour son utilisation pour favoriser la cicatrisation et/ou pour la prévention ou le traitement des infections microbiennes.

6. Composition pharmaceutique pour son utilisation selon la revendication 5, contenant au moins un autre principe actif.

## Patentansprüche

1. Verbindung der allgemeinen Formel I

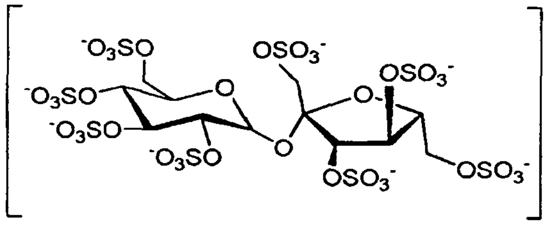 $[n\ Mg^{2+} + (8 - 2n)\ (MgY)^{+}]$

Formel I

wobei:

$0 \leq n \leq 4$
n eine ganze Zahl ist
Y OH, Cl, Br, I, $NO_3$, $BF_4$, $C_6H_5O_7$, $CH_3CO_2$, $CF_3CO_2$ oder $-OCH_3$ bedeutet,
zur Verwendung zur Begünstigung der Wundheilung und/oder zur Prävention oder Behandlung mikrobieller Infektionen.

**2.** Verbindung zur Verwendung nach Anspruch 1, **dadurch gekennzeichnet, dass** n = 4.

**3.** Verbindung zur Verwendung nach Anspruch 1 oder 2, **dadurch gekennzeichnet, dass** es sich um die Wundheilung von Verbrennungen und akuten oder chronischen Wunden handelt.

**4.** Verbindung zur Verwendung nach Anspruch 3, **dadurch gekennzeichnet, dass** die Verbrennungen und akuten oder chronischen Wunden auf eine Verbrennung durch Hitze oder durch einen Sonnenbrand, eine Strahlungsdermatitis, eine Reizung unterschiedlichen Ursprungs, eine Dermatitis, eine Abschürfung, eine Schramme, einen Kratzer, einen Schnitt, ein Geschwür, z.B. offenes Bein oder Magengeschwür, eine wund gelegene Stelle, eine durch Diabetes verursachte Wunde, eine Aphthe, eine Wunde des Mundraums unterschiedlichen Ursprungs, Aknenarben, eine Kryotherapienarbe, eine postchirurgische Narbe oder eine Narbe nach ästhetischer Dermatologie, eine Brandblase, eine Cheilitis, ein Ekzem, ein Gesäßmuskelerythem oder eine Dermatoporose zurückzuführen sind.

**5.** Arzneimittel, umfassend mindestens eine Verbindung wie in Anspruch 1 oder 2 definiert, und mindestens einen pharmazeutisch verträglichen Exzipienten zur Verwendung zur Begünstigung der Wundheilung und/oder zur Prävention oder Behandlung mikrobieller Infektionen.

**6.** Arzneimittel zur Verwendung nach Anspruch 5, welches mindestens einen anderen Wirkstoff enthält.

## Claims

**1.** A compound of general formula I

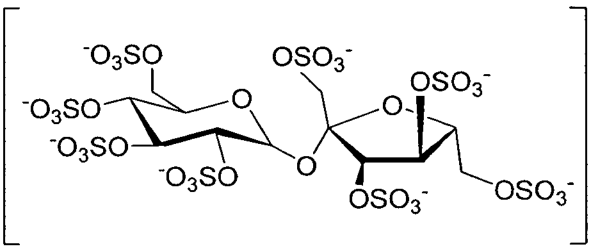 $[n\ Mg^{2+} + (8 - 2n)\ (MgY)^{+}]$

Formula I

wherein:

$0 \leq n \leq 4$
n is an integer
Y represents OH, Cl, Br, I, $NO_3$, $BF_4$, $C_6H_5O_7$, $CH_3CO_2$, $CF_3CO_2$ or $-OCH_3$, for use to promote cicatrization and/or for preventing or treating microbial infections.

2. The compound for use according to claim 1, **characterized in that** n=4.

3. The compound for use according to claim 1 or 2, **characterized in that** it is the cicatrization of burns and acute or chronic wounds.

4. The compound for use according to claim 3, **characterized in that** the burns and acute or chronic wounds are due to a burn by heat or sunburn, radiodermatitis, irritation of diverse origin, dermatitis, abrasions, scrapes, scratches, cuts, ulcers, for example of the leg or gastric, bedsores, wounds due to diabetes, mouth ulcers, wounds of the oral cavity of various origin, acne scars, cryotherapy scars, scars resulting from surgery or cosmetic dermatology, blisters, cheilitis, eczema, diaper rash or dermatoporosis.

5. A pharmaceutical composition comprising at least a compound as defined in claim 1 or 2, and at least a pharmaceutically acceptable excipient, for use to promote cicatrization and/or for preventing or treating microbial infections.

6. The pharmaceutical composition for use according to claim 5, containing at least one other active ingredient.

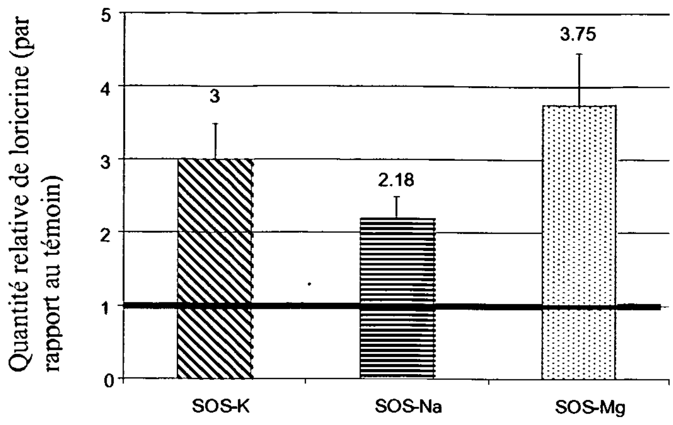


**Figure 1**

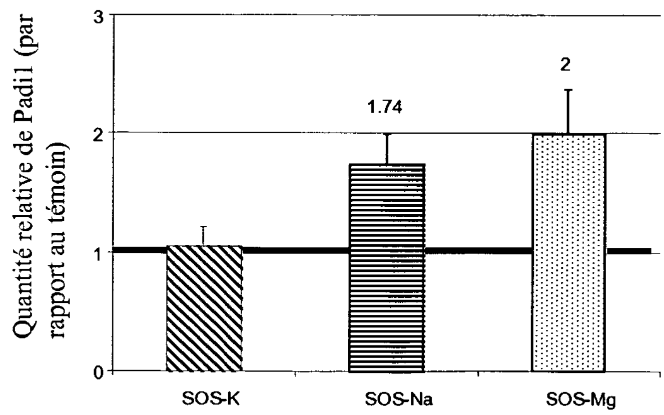


**Figure 2**

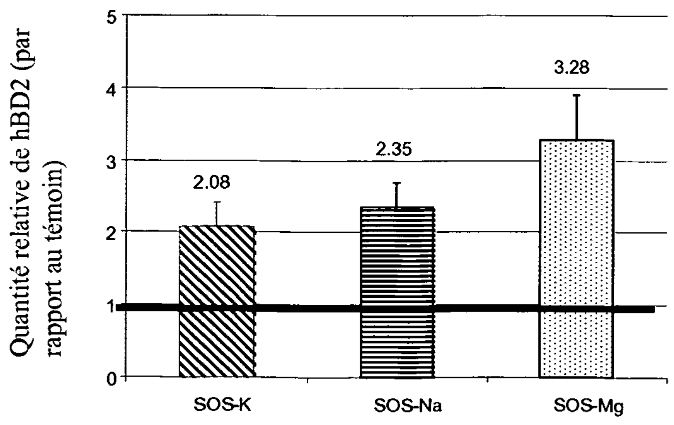


**Figure 3**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- WO 2006017752 A
- US 5767104 A
- FR 2646604
- WO 19940047 A
- FR 1390007
- EP 0230023 A
- FR 2916355
- US 5908836 A
- US 4581221 A
- EP 0640346 A